# EUROPEAN PATENT APPLICATION

(11) **EP 4 227 420 A1**
(43) Date of publication of application: **16.08.2023**
(21) Application number: 23156284.4
(22) Date of filing: 13.02.2023
(51) Int. Cl.: C12Q 1/00, C12Q 1/26, G01N 27/327, G01N 33/66

(54) **ELECTRODE FOR MEASURING GLUCOSE AND ELECTROCHEMICAL SENSOR INCLUDING THE SAME**

(30) Priority: 14.02.2022 JP 2022020449
(71) Applicant: ARKRAY, Inc., Kyoto-shi, Kyoto 601-8045 (JP)
(72) Inventor: MATSUMOTO, Kohei, Kyoto, 602-0008 (JP)
(74) Representative: Dehns

(57) **Abstract**

The present invention provides an electrode including: an electrode layer comprising an electrode material and a glucose oxidoreductase; a permeation restricting layer covering the enzyme electrode layer; and a glucose diffusion layer comprising a hydrophilic substance and disposed between the enzyme electrode layer and the permeation restricting layer.

## Description

### TECHNICAL FIELD

The present invention relates to an electrode for measuring glucose and an electrochemical sensor (glucose sensor) including the same.

### BACKGROUND ART

In a continuous glucose monitoring system using a conventional electrochemical sensor, an enzyme electrode having at least a four-layer structure including a substrate, an electrode layer, an enzyme layer and a permeation restricting layer is used. For example, Patent Document 1 discloses an electrochemical analyte sensor including an electrode and an analyte detection membrane, the analyte detection membrane including an obstacle layer and an enzyme layer, wherein the electrochemical analyte sensor includes a hydrophilic polymer layer disposed between the electrode and the obstacle layer or between the obstacle layer and the enzyme layer.

Further, Patent Document 2 discloses a biosensor characterized in that a hydrophilic polymer membrane containing at least one of an enzyme and an enzyme substrate is formed on the surface of an electrode composed of a hydrophobic material, and further discloses that the surface is covered by a permeation restricting membrane.

### PRIOR ART DOCUMENTS

### PATENT DOCUMENTS

[Patent Document 1] WO2010/107941
[Patent Document 2] JP2002-221508A

### SUMMARY OF THE INVENTION

In blood glucose level monitoring using a glucose sensor, it is important to stably monitor the blood glucose level accurately and continuously. Thus, glucose sensors have been required to achieve a further improvement in the linearity of the detected current value with respect to the glucose concentration, as well as an improvement in the stability of the electrode so as to prevent a decrease in the detected current value and the linearity even during long-term monitoring.

Therefore, the present inventors conducted an evaluation of a conventional glucose sensor including an electrode that includes an enzyme electrode layer and a permeation restricting layer covering the same, to determine that the linearity of the response current value decreases as the glucose concentration increases, and further, that the response current value gradually decreases during long-term monitoring.

Accordingly, an object of the present invention is to improve a conventional electrode that includes an enzyme electrode layer and a permeation restricting layer covering the same, and to provide an enzyme electrode which allows for improving the linearity between the glucose concentration and the response current value, and which is capable of stably performing continuous monitoring, and a glucose sensor including the same.

The present inventors have conducted an examination in order to solve the above-mentioned problems.

First, the present inventors have investigated the cause of a decrease in the linearity of the response current value and a decrease in the stability in a conventional electrode that includes an enzyme electrode layer and a permeation restricting layer covering the same, and formulated a hypothesis that the cause of the decrease in the linearity of the response current value is the fact that only some of the enzyme molecules in the enzyme electrode layer are utilized in the reaction due to the non-uniformity of the polymer structure of the permeation restricting layer. As a result of endeavours to improve the problems arising from the non-uniformity of the permeation restricting layer, under the hypothesis described above, the inventors have found out the following: by providing a glucose diffusion layer containing a hydrophilic substance between an enzyme electrode layer containing an electrode material and a glucose oxidoreductase, and a permeation restricting layer covering the enzyme electrode layer, glucose diffuses uniformly in the glucose diffusion layer, making it possible for substantially all enzyme molecules in the enzyme electrode layer to be utilized in the reaction, resulting in an improvement in the linearity between the glucose concentration and the response current value. The inventors have also found out that providing the glucose diffusion layer between the enzyme electrode layer and the permeation restricting layer enables the electrode to obtain a stable response current value for a long period of time, and that the above-described electrode can be used in a glucose sensor capable of performing continuous monitoring accurately and stably, thereby completing the present invention.

As described above, Patent Document 1 discloses an electrochemical analyte sensor including an electrode and an analyte sensing membrane, the analyte sensing membrane including an interference layer and an enzyme layer, wherein the electrochemical analyte sensor includes a hydrophilic polymer layer disposed between the electrode and the obstacle layer or between the obstacle layer and the enzyme layer. However, Patent Document 1 discloses only the fact that the hydrophilic polymer layer is provided on the side of the electrode and is silent about providing the hydrophilic polymer layer on the side of a permeation restricting membrane. It does not disclose or suggest the idea of uniformly diffusing glucose that has permeated through the permeation restricting membrane in the enzyme layer. On the contrary, this sensor is based on the premise that an obstacle layer is present.

Further, Patent Document 2 discloses, as described above, a biosensor characterized in that a hydrophilic polymer membrane containing at least one of an enzyme and an enzyme substrate is formed on the surface of an electrode composed of a hydrophobic material, and further discloses that the surface is covered by a permeation restricting membrane. However, there is a problem that the electron transmission is inhibited when the enzyme and the hydrophilic polymer are mixed in the same layer, causing a decrease in the measured current value.

One embodiment according to the present invention provides an electrode comprising:
an enzyme electrode layer comprising an electrode material and a glucose oxidoreductase;
a permeation restricting layer covering the enzyme electrode layer; and
a glucose diffusion layer comprising a hydrophilic substance and disposed between the enzyme electrode layer and the permeation restricting layer.

In one embodiment of the electrode, the enzyme electrode layer has a layer structure including a layer of a metal or carbon as the electrode material, and a layer containing the glucose oxidoreductase disposed on the layer of a metal or carbon.

In another embodiment of the electrode, the enzyme electrode layer is a layer containing a mixture of an electrically conductive substance as the electrode material and the glucose oxidoreductase.

Another embodiment according to the present invention provides a glucose sensor including the electrode.

Still another embodiment according to the present invention provides a method of producing an electrode, for example an electrode as herein described, the method comprising the steps of:
forming an enzyme electrode layer comprising an electrode material and a glucose oxidoreductase;
laminating a glucose diffusion layer comprising a hydrophilic substance on the enzyme electrode layer; and
laminating a permeation restricting layer on the glucose diffusion layer.

### EFFECTS OF THE INVENTION

In the electrode according to the present invention, the glucose that has passed through the permeation restricting layer can be uniformly diffused by providing a glucose diffusion layer containing a hydrophilic substance between the permeation restricting layer and the enzyme electrode layer, making it possible to uniformly supply glucose to substantially all enzyme molecules in the enzyme electrode layer. As a result, the linearity of the response current value with respect to the glucose concentration is improved. Further, in the electrode according to the present invention, a high response current value can be maintained for a long period of time, by providing the glucose diffusion layer containing a hydrophilic substance between the permeation restricting layer and the enzyme electrode layer. Therefore, the electrode according to the present invention allows for producing a glucose sensor capable of performing continuous monitoring accurately and for an extended time.

### BRIEF DESCRIPTION OF THE DRAWINGS

FIG. 1 is a schematic diagram showing one embodiment of an electrode according to the present invention.
FIG. 2 is a diagram showing one embodiment (continuous glucose monitoring device A) of a glucose measurement system including a glucose sensor according to the present invention.
FIG. 3 is a graph showing the results of plotting the glucose response current values obtained using a glucose sensor including the electrode of Example 1 or Comparative Example 1.
FIG. 4 is a graph showing the changes over time in the glucose response current values obtained using a glucose sensor including the electrode of Example 1 or Comparative Example 1.
FIG. 5 is a graph showing the results of plotting the glucose response current values obtained using a glucose sensor including any one of the electrodes of Examples 1 to 4.
FIG. 6 is a graph showing the results of plotting the glucose response current values obtained using a glucose sensor including the electrode of Example 5 or Comparative Example 1.

### EMBODIMENTS FOR CARRYING OUT THE INVENTION

### < Electrode >

The electrode according to the present invention comprises:
an enzyme electrode layer comprising an electrode material and a glucose oxidoreductase;
a permeation restricting layer covering the enzyme electrode layer; and
a glucose diffusion layer comprising a hydrophilic substance and disposed between the enzyme electrode layer and the permeation restricting layer.

### < Electrode Material >

The electrode material may be, for example, a metallic material or a carbon material. Examples of the metallic material include gold (Au), platinum (Pt), silver (Ag) and palladium (Pd). Examples of the carbon material include carbon, and examples of the carbon include graphite, carbon nanotubes, graphene and mesoporous carbon.

### < Glucose Oxidoreductase >

The glucose oxidoreductase can be any enzyme capable of oxidizing and reducing glucose which is the target substance to be measured. Examples of the glucose oxidoreductase include glucose dehydrogenase (GDH) and glucose oxidase. The glucose oxidoreductase preferably contains at least one of pyrroloquinoline quinone (PQQ) and flavin adenine dinucleotide (FAD), as a catalytic subunit or a catalytic domain. Examples of the glucose oxidoreductase containing PQQ include PQQ glucose dehydrogenase (PQQGDH). Examples of the glucose oxidoreductase containing FAD include cytochrome glucose dehydrogenase (CyGDH) and glucose oxidase (GOD) which have an α-subunit containing FAD.

Further, the glucose oxidoreductase can contain an electron transfer subunit or an electron transfer domain. The electron transfer subunit may be, for example, a subunit containing heme which has a function of giving and receiving electrons. The oxidoreductase having the subunit containing the heme may be, for example, glucose dehydrogenase containing cytochrome, or a fusion protein (for example as described in WO2005/030807) of PQQGDH with cytochrome.

### < Enzyme Electrode Layer>

The enzyme electrode layer contains the electrode material and the glucose oxidoreductase as described above.

The enzyme electrode layer may contain other optional components in order to allow the oxidation-reduction reaction of glucose to proceed efficiently. The other optional component(s) may be, for example, one or more components selected from the group consisting of an electrically conductive substance, a cross-linking agent and a saccharide.

The electrically conductive substance may be, for example, an electrically conductive polymer or electrically conductive particles.

The electrically conductive polymer is preferably one which is soluble in water, and examples thereof include polyacetylene, polyparaphenylene, polyaniline, polythiophene, polyparaphenylene vinylene, polypyrrole, polyacene, graphene and polyethylenedioxythiophene.

The electrically conductive particles may be, for example, metal particles or particles of a higher-order structure made of carbon. Examples of the metal particles include particles of gold, silver, copper, nickel, vanadium, platinum, palladium, iron, aluminum, titanium, zinc, tin, tungsten and chromium. Examples of the particles of the higher-order structure made of carbon include carbon particles and fine carbon particles, such as particles of electrically conductive carbon black, carbon nanotubes (CNTs) and fullerene. Examples of the electrically conductive carbon black include oil furnace black, Ketjen Black, Black Pearls and acetylene black.

The enzyme electrode layer can also contain a cross-linking agent. The type of the cross-linking agent is not particularly limited as long as the agent is capable of cross-linking the enzyme or the electrically conductive substance. The cross-linking agent may be, for example, a compound containing at least one functional group selected from the group consisting of an aldehyde group, a maleimide group, a carbodiimide group, an oxazoline group and an epoxy group. The form of the cross-linking agent is not limited and the cross-linking agent may be in the form of either a monomer or a polymer.

The enzyme electrode layer can also contain a saccharide. The type of the saccharide is not particularly limited as long as the saccharide is capable of stabilizing the glucose oxidoreductase. Examples of the saccharide include trehalose, sucrose, maltose, mannose, fructose, lactose, maltotriose and raffinose.

According to one embodiment of the electrode of the present invention, the enzyme electrode layer has a layer structure including a layer of a metal or carbon as the electrode material, and a layer containing the glucose oxidoreductase disposed on the layer of a metal or carbon.

For example, the enzyme electrode layer can be formed by forming a layer of a metal or carbon (electrode material) on a substrate, and forming a layer (also referred to as an "enzyme layer" or a "reagent layer") containing the glucose oxidoreductase on the layer of a metal or carbon.

According to another embodiment of the electrode of the present invention, the enzyme electrode layer is a layer containing a mixture of an electrically conductive substance as the electrode material and the glucose oxidoreductase.

For example, the enzyme electrode layer can be formed by mixing reagents containing the glucose oxidoreductase, the electrically conductive substance and carbon (electrode material), and coating the mixture on a substrate.

### < Substrate >

The electrode is preferably provided on a substrate. A substrate commonly used in an electrochemical sensor can be used as the substrate. The substrate may be, for example, a substrate made of a thermoplastic resin, any of various types of resins (plastics) or an insulating material. Examples of the thermoplastic resin include polyetherimide (PEI), polyether ether ketone (PEEK), polyethylene terephthalate (PET) and polyethylene (PE). Examples of the various types of resins (plastics) include polyimide resins and epoxy resins. Examples of the insulating material include glasses, ceramics and papers. Among these, a substrate made of an insulating material is preferred.

### < Permeation Restricting Layer >

The permeation restricting layer covers the enzyme electrode layer. The permeation restricting layer is provided for restricting the permeation of glucose into the glucose diffusion layer and the enzyme electrode layer to adjust the amount of glucose that reaches the glucose diffusion layer and the enzyme electrode layer, in order to prevent the sensor sensitivity from saturating too quickly, namely, to prevent the sensitivity from saturating, until the upper limit of the concentration of glucose detectable by the sensor is reached.

The permeation restricting layer is made of a material which is capable of allowing glucose to pass therethrough while restricting the amount thereof, and is preferably made of an insoluble material. For example, the permeation restricting layer is made of a polymer or a porous material containing a hydrophilic group.

Specific examples of such a material include polyethylene-co-tetrafluoroethylene, polyolefins, polyesters, polycarbonates, polytetrafluoroethylene, homopolymers, copolymers and terpolymers of polytetrafluoroethylene, polypropylene, polyvinyl chloride, polyvinylidene fluoride, polybutylene terephthalate, polymethyl methacrylate, polyether ether ketone, polyurethane, cellulose polymers, polysulfones, silicon-based polymers (polysiloxanes), as well as cellulose acetate, HEMA (hydroxyethyl methacrylate) and copolymers containing these compounds, and polyvinyl pyridine.

Among these, one or more materials selected from the group consisting of a cellulose polymer, polyurethane and polyvinyl pyridine are preferred.

The permeation restricting layer may have any thickness as long as the layer allows glucose to permeate therethrough while restricting the amount thereof. For example, the permeation restricting layer preferably has a thickness of 1 µm or more and 100 µm or less, and more preferably 5 µm or more and 50 µm or less.

### < Glucose Diffusion Layer >

The glucose diffusion layer is present between the permeation restricting layer and the enzyme electrode layer so as to allow the glucose that has passed through the permeation restricting layer to diffuse and to be uniformly supplied to the enzyme electrode layer. The glucose diffusion layer contains a hydrophilic substance. The glucose diffusion layer has a better ability to diffuse glucose as compared to the permeation restricting layer, due to the presence of a hydrophilic substance.

Since glucose does not pass through the permeation restricting layer uniformly, the region of the electrode surface to which glucose reaches is limited in the case of a conventional electrode that includes the permeation restricting layer alone. This is thought to cause a phenomenon in which only some of the enzyme molecules in the enzyme electrode can be utilized in the reaction, leading to a decrease in the linearity of the response current value with respect to the glucose concentration.

In the case of using a polymer such as polyurethane as the material of the permeation restricting layer, for example, the polymer such as polyurethane contains hydrophilic moieties and hydrophobic moieties in an irregular manner, and thus, glucose is believed to pass through the moieties with a high hydrophilicity in the polymer. When the permeation restricting layer using such a polymer is formed directly on the enzyme layer, glucose passes only through the moieties of the polymer with a high hydrophilicity, failing to diffuse over the entire enzyme electrode layer, and is able to react only with some of the enzyme molecules in the enzyme electrode layer.

By including a glucose diffusion layer containing a hydrophilic substance between the permeation restricting layer and the enzyme electrode layer, in contrast, the electrode according to the present invention is capable of allowing the glucose that has passed through the permeation restricting layer to diffuse uniformly within the glucose diffusion layer. As a result, it becomes possible for glucose to reach the entire enzyme electrode layer uniformly, thereby enabling an improvement in the linearity of the response current value with respect to the glucose concentration.

In order to obtain such an effect, the glucose diffusion layer is preferably provided so as to cover the entire surface of the enzyme electrode layer.

The hydrophilic substance is not particularly limited as long as the substance is capable of diffusing glucose. The hydrophilic substance may be, for example, a hydrophilic polymer or any other hydrophilic substance. Examples of the hydrophilic polymer include: polyvinylpyrrolidone (PVP), poly-N-vinyl-3-ethyl-2-pyrrolidone, poly-N-vinyl-4,5-dimethyl-2-pyrrolidone, polyacrylamide, poly-N,N-dimethylacrylamide, polyvinyl alcohol, polyethylene glycol, polymers containing a pendant ionic group, and copolymers thereof and compounds containing these polymers. Examples of the other hydrophilic substance include hydrophilic substances other than hydrophilic polymers, such as sodium alginate, methyl acrylate, chitosan, carboxyl methyl cellulose, carboxyl ethyl cellulose and sodium acrylate.

Among these polymers, one or more selected from the group consisting of polyethylene glycol, polyvinylpyrrolidone, polyvinyl alcohol and sodium alginate are preferred.

The hydrophilic substance in the glucose diffusion layer is preferably subjected to a cross-linking treatment, so that the hydrophilic substance is not dissolved by a sample liquid.

The cross-linking treatment can be carried out, for example, with a cross-linking agent.

The type of the cross-linking agent is not particularly limited as long as the agent is a substance capable of cross-linking the hydrophilic substance so as to prevent it from dissolving in a sample liquid, and is selected as appropriate depending on the type of the hydrophilic substance. Examples of the cross-linking agent include cross-linking agents capable of targeting (i.e. reacting with) a carboxyl group as the cross-linking target, such as cross-linking agents containing an oxazoline group, cross-linking agents containing an epoxy group and cross-linking agents containing a carbodiimide group.

For example, by mixing a cross-linking agent and a hydrophilic substance to prepare an aqueous solution, coating the aqueous solution on the enzyme electrode layer, and then allowing a cross-linking reaction to proceed by heating, UV irradiation or the like, it is possible to form a glucose diffusion layer containing the hydrophilic substance that has been subjected to a cross-linking treatment, on the enzyme electrode layer.

The amount of the hydrophilic substance in the glucose diffusion layer may be any amount as long as it is enough for sufficiently diffusing glucose. For example, the amount of the hydrophilic substance is preferably 1.19 µg/cm² or more and 1.19 g/cm² or less, more preferably 6 µg/cm² or more and 119 mg/cm² or less, and still more preferably 11.9 µg/cm² or more and 11.9 mg/cm² or less.

The glucose diffusion layer may have any thickness as long as it is enough for sufficiently diffusing glucose within the layer. For example, the glucose diffusion layer preferably has a thickness of 10 nm or more and 10 mm or less, more preferably 50 nm or more and 1 mm or less, and still more preferably 100 nm or more and 100 µm or less.

### < Example of Electrode >

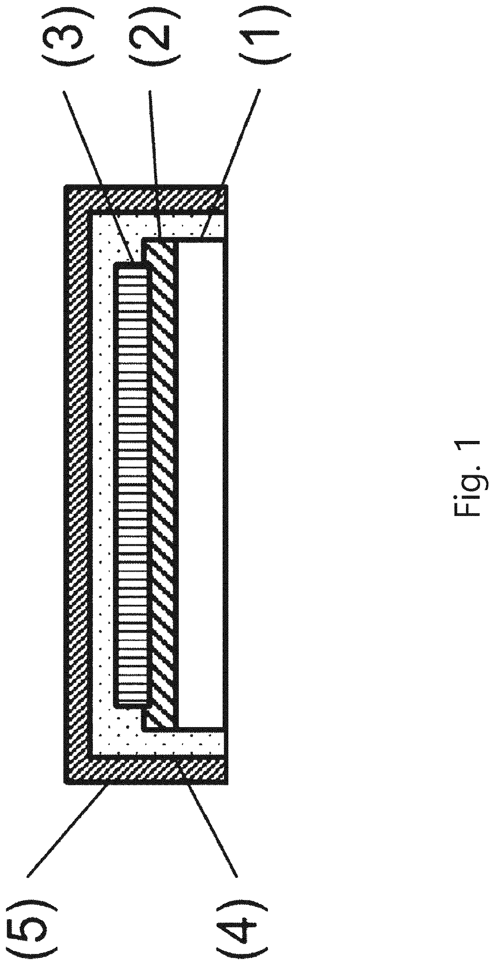

An electrode according to one embodiment of the present invention will be described with reference to FIG. 1.

An electrode layer (2) composed of an electrode material such as a metal or carbon is formed on a substrate (1), and an enzyme layer (3) containing a glucose oxidoreductase is formed on the electrode layer (2).

Further, a glucose diffusion layer (4) covering the enzyme layer (3) is provided, and a permeation restricting layer (5) covering the glucose diffusion layer (4) is provided. The enzyme layer (3) may contain any of the electrically conductive substance, the cross-linking agent and the saccharide as described above, in addition to the glucose oxidoreductase.

The glucose diffusion layer (4) is preferably provided so as to cover the entire enzyme layer (3), and may be provided only on the enzyme layer (3).

In cases where the enzyme and the electrode material are mixed to form an enzyme electrode layer on the substrate, one layer of the enzyme electrode layer containing the enzyme and the electrode material is formed instead of the electrode layer (2) and the enzyme layer (3), and on the enzyme electrode layer, the glucose diffusion layer is formed, and the permeation restricting layer is further formed. In this case, the enzyme electrode layer may contain any of the electrically conductive substance, the cross-linking agent and the saccharide as described above, in addition to the electrode material and the glucose oxidoreductase. Further, the glucose diffusion layer (4) is preferably provided so as to cover the entire enzyme electrode layer, and may be provided only on the enzyme electrode layer.

When a liquid sample containing glucose is added onto the permeation restricting layer (5), the glucose permeates through the permeation restricting layer (5) and reaches the glucose diffusion layer (4). The glucose is uniformly diffused within the glucose diffusion layer (4) due to the presence of the hydrophilic substance, and uniformly reaches substantially all glucose oxidoreductase molecules within the enzyme layer (3) to cause an oxidation (reduction) reaction. Electrons generated by this reaction are transferred to the electrode layer (2), and a current that accurately reflects the glucose concentration in the liquid sample flows by applying a voltage to the electrodes. In cases where the glucose oxidoreductase and the electrode material are mixed to form one enzyme electrode layer, the glucose that has diffused uniformly due to the presence of the hydrophilic substance within the glucose diffusion layer (4) uniformly reaches the glucose oxidoreductase molecules within the enzyme electrode layer to cause an oxidation (reduction) reaction. Electrons generated by this reaction are transferred to the electrode material in the enzyme electrode layer, and a current that accurately reflects the glucose concentration in the liquid sample flows by applying a voltage to the electrodes.

### < Example of Method of Preparing Electrode >

The electrode as described is prepared, for example, as follows. Specifically, a layer of a metal, carbon or the like as an electrode material is formed on one surface of an insulating substrate. For example, a carbon film having a desired thickness (for example, about 10 µm) can be formed by screen printing a carbon ink on one surface of a film-like insulating substrate having a predetermined thickness (for example, about 100 µm). It is also possible to form a metal layer having a desired thickness (for example, about 30 nm), instead of the carbon layer, by forming a film by the physical vapor deposition (PVD, such as sputtering) or the chemical vapor deposition (CVD) of a metallic material.

Next, an enzyme layer is formed on the electrode. For example, a solution containing an oxidoreductase and an electrically conductive substance is prepared, and the solution is dropped on the surface of the electrode. The solution is dried and solidified on the electrode to form an enzyme layer on the electrode.

Subsequently, a glucose diffusion layer containing a hydrophilic substance is formed on the enzyme layer. For example, a solution of a hydrophilic substance can be coated on the enzyme layer and then dried to form a glucose diffusion layer containing the hydrophilic substance on the enzyme layer. Alternatively, a solution of a hydrophilic substance can be used to perform spin coating, dip coating, drop coating, or the like, to form a glucose diffusion layer containing the hydrophilic substance. In the glucose diffusion layer, the hydrophilic substance is preferably subjected to a cross-linking treatment.

Thereafter, a permeation restricting layer is formed on the glucose diffusion layer. For example, a solution of a cellulose polymer, polyurethane, polyvinyl pyridine and/or the like can be coated on the glucose diffusion layer and then dried to form a permeation restricting layer on the glucose diffusion layer. Alternatively, a solution of a cellulose polymer, polyurethane, polyvinyl pyridine and/or the like can be used to perform spin coating, dip coating, drop coating, or the like, to form a permeation restricting layer.

### < Glucose Sensor >

The electrode according to the present invention can be used as a working electrode of a glucose sensor. That is, the glucose sensor according to the present invention includes the electrode according to the present invention, and preferably, further includes an electrode that serves as a counter electrode. The counter electrode may be any electrode that can be commonly used as a counter electrode of a glucose sensor. For example, a silver/silver chloride electrode or a carbon electrode can be used as the counter electrode. The counter electrode, as well, can be formed by forming a film on a substrate, by physical vapor deposition (PVD, such as sputtering), chemical vapor deposition (CVD) or screen printing. The glucose sensor may be configured to have a three-electrode system which further includes a reference electrode.

The glucose sensor can be produced by forming the electrode according to the present invention that serves as the working electrode, and a silver/silver chloride electrode or a carbon electrode that serves as the counter electrode, on a substrate.

The glucose sensor including the electrode according to the present invention may be a body-implantable sensor, a batch type sensor, or a sensor to be used when necessary.

The glucose sensor is preferably a sensor for continuous glucose monitoring, and a body-implantable sensor which is placed, for example, subcutaneously.

By using the glucose sensor according to the present invention, it is possible to measure the concentration of glucose contained in a sample. The sample is not particularly limited as long as the sample contains glucose, and may be, for example, a biological sample. Examples of the biological sample include blood, interstitial fluid, plasma, lymph fluid, cerebrospinal fluid, urine, sweat and saliva. Among these, blood, interstitial fluid or plasma are preferred.

### < Method of Measuring Glucose >

A method of measuring glucose according to the present invention includes the steps of:
bringing a sample containing glucose into contact with a glucose sensor as herein described;
applying a voltage to the electrodes of the glucose sensor to measure the value of the current that flows due to the oxidation-reduction reaction of glucose by a glucose oxidoreductase; and
calculating the glucose concentration based on the measured current value.

The step of bringing a sample containing glucose into contact with a glucose sensor can be carried out by bringing the sample into contact with the surface of the permeation restricting layer of the electrode of the glucose sensor. In the case of a body-implantable sensor, an embodiment in which the sensor is implanted in the body so that the sensor is in a state where a sample such as blood or interstitial fluid is in contact with the permeation restricting layer of the electrode of the glucose sensor, is also included.

By bringing the sample containing glucose into contact with the surface of the permeation restricting layer of the glucose sensor, the glucose in the sample passes through the permeation restricting layer and is diffused in the diffusion layer, and uniformly reaches the enzyme electrode layer. The glucose oxidoreductase causes the oxidation reaction of glucose, and electrons are transferred to the electrode. By applying a potential to the electrode, a current corresponding to the amount of glucose flows between the electrodes. Since the current value varies depending on the concentration of glucose in the sample, the glucose concentration in the sample can be calculated based on the measured current value.

The voltage to be applied to the electrodes is preferably from 0 mV to 800 mV, and more preferably from 40 mV to 200 mV, for example, with respect to a silver/silver chloride electrode as the reference electrode.

### < Glucose Measurement System>

A glucose measurement system according to one embodiment of the present invention includes at least:
a glucose sensor that includes the electrode according to the present invention;
a power supply configured to apply a voltage to the electrodes of the glucose sensor;
a detection unit configured to detect a response current; and
an arithmetic unit configured to calculate the concentration of glucose from the response current value.

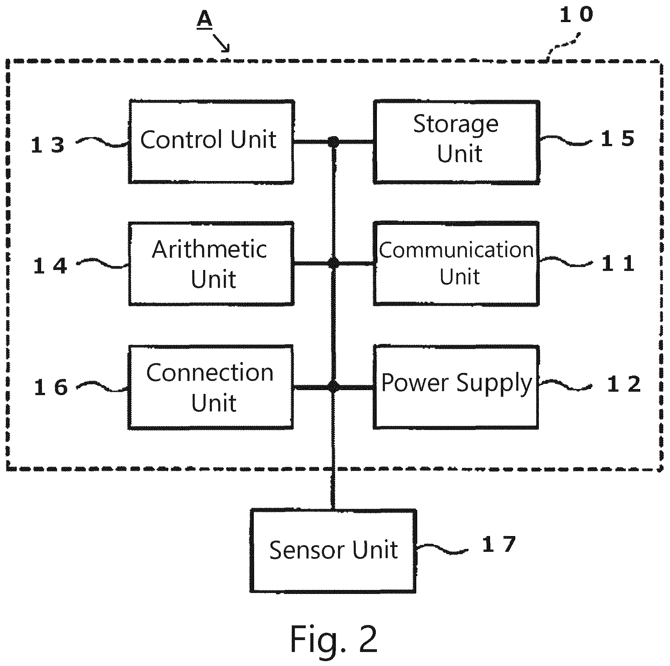

One embodiment of the glucose measurement system (a continuous glucose monitoring device A) including the glucose sensor according to the present invention will be described with reference to FIG. 2.

As shown in FIG. 2, a circuit board 10 includes: a communication unit 11, a power supply 12, a control unit 13, an arithmetic unit 14, a storage unit 15 and a connection unit 16.

The connection unit 16 is electrically connected to the electrode according to the present invention, namely, to the enzyme electrode layer of a sensor unit 17, and is used for applying a voltage to the sensor unit 17 to obtain a response current. The sensor unit 17 corresponds to the glucose sensor according to the present invention.

The communication unit 11 performs data communication between the continuous glucose monitoring device A and an external information processing device. The communication unit 11 includes at least a transmission unit, and includes a reception unit as necessary. A wireless communication means such as infrared communication or Bluetooth (registered trademark), for example, can be used as a means for data communication. Further, the communication unit 11 may also be configured to perform data communication by wire. In this case, a cable connecting portion is provided to the communication unit 11 of the continuous glucose monitoring device A, and the cable connecting portion is connected with an external information processing device, or the like, via a cable to perform data communication.

The external information processing device may be, for example, an insulin delivery device that administers insulin to the human body, a simple blood glucose level measuring device, a watch-type display device or a personal computer.

The power supply 12 is a direct current power supply for supplying power to the circuit board 10 and the sensor unit 17. For example, a battery with a power supply voltage of from 1 to 3 V can be used as the power supply 12.

The control unit 13 controls the entire continuous glucose monitoring device A. For example, the control unit 13 controls the timing for applying a voltage, the sampling of the response current, the arithmetic processing of the glucose concentration, the communication with the external information processing device, and the like.

The arithmetic unit 14 performs various arithmetic operations required for the processing in the continuous glucose monitoring device A, including, for example, the calculation of the glucose concentration.

The storage unit 15 stores various types of programs to be executed in the control unit 13, for example, a program for executing a test mode processing routine to be described later, as well as data such as calibration curves to be used in the arithmetic operations in the arithmetic unit 14, correction data and applied voltage patterns. Further, the storage unit 15 stores the data of the response current detected by the sensor unit 17, and the data of the glucose concentration calculated in the arithmetic unit 14.

The control unit 13, the arithmetic unit 14 and the storage unit 15 include electronic parts, such as a CPU or MPU, a ROM and a RAM mounted on the circuit board 10.

### EXAMPLES

The present invention will now be described specifically, with reference to Examples. However, the present invention is in no way limited to the embodiments of the following Examples.

### < Production Example of Electrode: Example 1 >

A metal (gold) was sputtered on a substrate made of polyether ether ketone to form a metal layer, and a part of the metal layer was removed by etching to form an electrode pattern.

On the resulting electrode (metal layer), 12 nL of a mixture of the following reagents (dissolved in water to achieve the respective final concentrations) was coated, dried and heated to form an enzyme layer (reagent layer).

| Reagent | Final concentration |
|---|---|
| Phosphate buffer | 10 mM |
| Trehalose | 0.43 wt% |
| GDH | 8.5 mg/mL |
| Cross-linking agent | 5% |
| Carbon black dispersion liquid | 0.4 wt% |

Subsequently, the portion of the electrode including the enzyme layer was immersed in a 6w/w% polyvinyl alcohol aqueous solution containing the cross-linking agent to cover the enzyme layer with polyvinyl alcohol, thereby forming a glucose diffusion layer on the enzyme layer. Further, the resultant layer was dried and heated to carry out the cross-linking treatment of the glucose diffusion layer.

Thereafter, the portion of the electrode including the enzyme layer covered by the glucose diffusion layer was immersed in a solution of polyurethane in dimethylformamide to cover the glucose diffusion layer with polyurethane, and to form a permeation restricting layer on the glucose diffusion layer, thereby producing an electrode.

### < Production Example of Electrode: Comparative Example 1 >

The same procedure as in Example 1 was repeated to produce an electrode, except that the glucose diffusion layer containing polyvinyl alcohol was not provided.

### < Evaluation of Electrode: Example 1 and Comparative Example 1 >

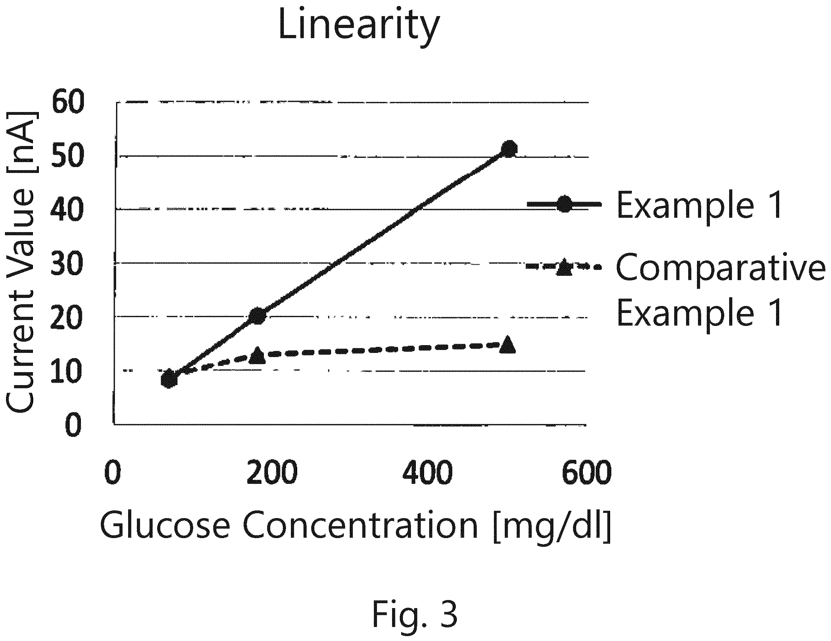

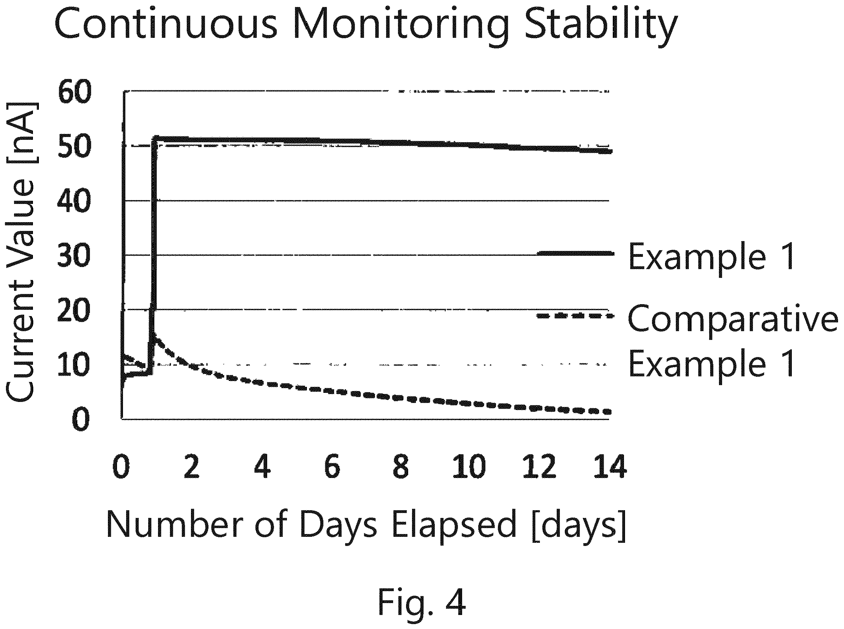

Using each of the electrodes of Example 1 and Comparative Example 1 obtained as described above, a phosphate buffered saline containing glucose as a sample was deposited onto the permeation restricting layer, then a voltage of 150 mV with respect to an Ag/AgCl reference electrode was applied, and the value of the current flowing between the electrodes was measured using an electrochemical measuring device. The measurement was started at a glucose concentration of 70 mg/dl, and the glucose concentration was raised to 180 mg/dl about 20 hours later, and raised to 500 mg/dl another hour later. Thereafter, the measurement was continued for 14 days. The plots of the response current values at glucose concentrations of 70, 180 and 500 mg/dl in this evaluation are shown in FIG. 3; and the changes over time in the response values during the 14-day measurement are shown in FIG. 4.

As can be seen from FIG. 3, a linear increase in the response current value was observed up to the high-concentration side of glucose, in Example 1. In Comparative Example 1, in contrast, a linear increase in the response value was no longer observed at the time point of a glucose concentration of 180 mg/dl.

As can be seen from FIG. 4, a decrease in the response current value of about 10% was observed with respect to the response value one day after the start of the measurement during the 14-day measurement, in Example 1, whereas a decrease of about 90% was observed in Comparative Example 1.

### < Examination on Concentration of Polyvinyl Alcohol in Glucose Diffusion Layer >

The same procedure as in Example 1 was repeated except that the glucose diffusion layer was formed with the concentration of the polyvinyl alcohol solution adjusted to about 4 w/w % to produce an electrode (Example 2).

The same procedure as in Example 1 was repeated except that the glucose diffusion layer was formed with the concentration of the polyvinyl alcohol solution adjusted to about 8 w/w % to produce an electrode (Example 3).

The same procedure as in Example 1 was repeated except that the glucose diffusion layer was formed with the concentration of the polyvinyl alcohol solution adjusted to about 10 w/w % to produce an electrode (Example 4).

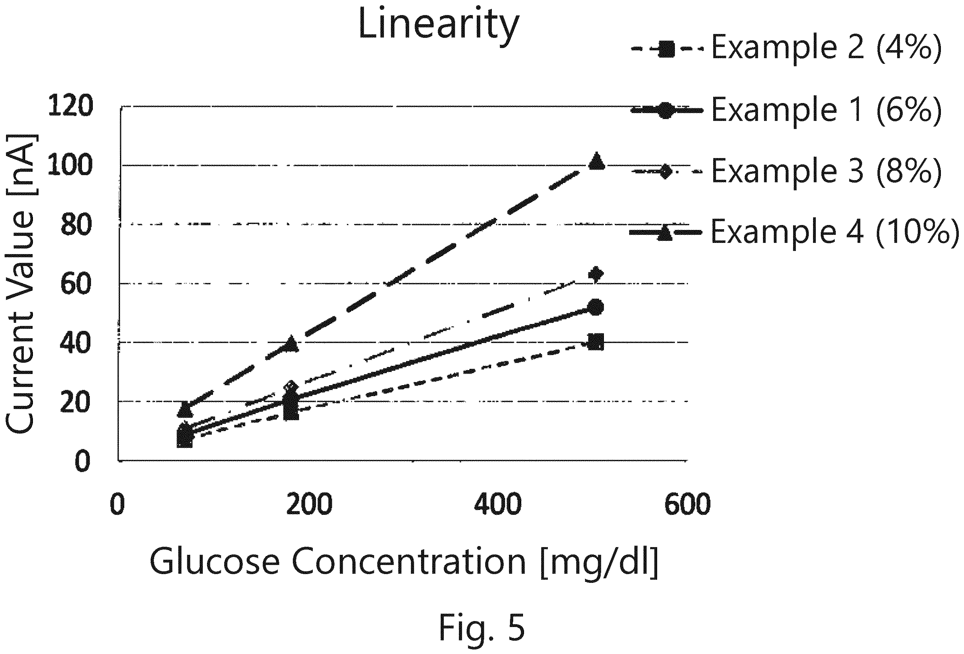

A phosphate buffered saline containing glucose as a sample was deposited onto the permeation restricting layer of each of the electrodes of Example 1 and Examples 2, 3 and 4, then a voltage of 150 mV with respect to the Ag/AgCl reference electrode was applied, and the value of the current flowing between the electrodes was measured using an electrochemical measuring device. The measurement was started at a glucose concentration of 70 mg/dl, and the glucose concentration was raised to 180 mg/dl about 20 hours later, and raised to 500 mg/dl another hour later. Thereafter, the measurement was continued for 14 days. The plots of the response values at glucose concentrations of 70, 180 and 500 mg/dl in this evaluation are shown in FIG. 5.

As can be seen from FIG. 5, the value of the response current to glucose improves as the concentration of polyvinyl alcohol used increases. This is assumed to be due to a greater diffusion of glucose as a result of an increase in the thickness of the glucose diffusion layer.

### < Example Using Sodium Alginate for Glucose Diffusion Layer >

The same procedure as in Example 1 was repeated except that 6 w/w % sodium alginate was used instead of polyvinyl alcohol as the material of a glucose diffusion layer, and that calcium chloride was used for cross-linking, to produce an electrode (Example 5).

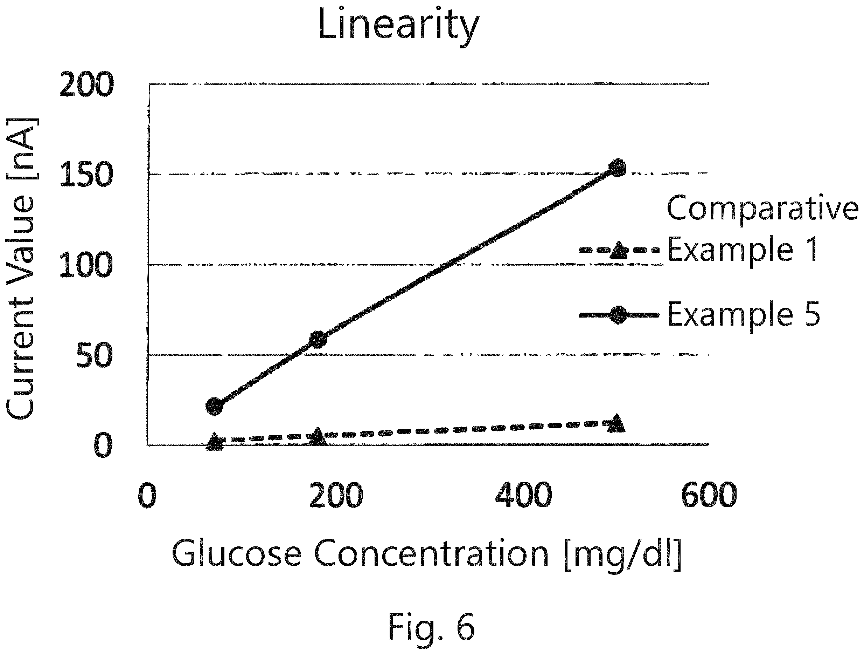

A phosphate buffered saline containing glucose as a sample was deposited onto the permeation restricting layer of each of the electrodes of Example 5 and Comparative Example 1, then a voltage of 150 mV with respect to the Ag/AgCl reference electrode was applied, and the value of the current flowing between the electrodes was measured using an electrochemical measuring device. The measurement was started at a glucose concentration of 70 mg/dl, and thereafter, the glucose concentration was raised to 180 mg/dl, and then raised to 500 mg/dl. The plots of the current values at glucose concentrations of 70, 180 and 500 mg/dl in this evaluation are shown in FIG. 6.

As can be seen from FIG. 6, the use of sodium alginate improves the value of the response current to glucose as compared to Comparative Example 1, and sodium alginate can also be used as the material of the glucose diffusion layer.

### DESCRIPTION OF SYMBOLS

- 1: substrate
- 2: electrode layer
- 3: enzyme layer
- 4: glucose diffusion layer
- 5: permeation restricting layer
- A: continuous glucose monitoring device
- 10: circuit board
- 11: communication unit
- 12: power supply
- 13: control unit
- 14: arithmetic unit
- 15: storage unit
- 16: connection unit
- 17: sensor unit

## Claims

1. An electrode comprising:
an enzyme electrode layer comprising an electrode material and a glucose oxidoreductase;
a permeation restricting layer covering the enzyme electrode layer; and
a glucose diffusion layer comprising a hydrophilic substance and disposed between the enzyme electrode layer and the permeation restricting layer.

2. The electrode according to claim 1, wherein the enzyme electrode layer has a layer structure comprising a layer of a metal or carbon as the electrode material, and a layer containing the glucose oxidoreductase disposed on the layer of a metal or carbon.

3. The electrode according to claim 1, wherein the enzyme electrode layer is a layer containing a mixture of an electrically conductive substance as the electrode material and the glucose oxidoreductase.

4. The electrode according to any one of claims 1 to 3, wherein the glucose oxidoreductase is glucose dehydrogenase.

5. The electrode according to any one of claims 1 to 4, wherein the hydrophilic substance is a hydrophilic polymer.

6. The electrode according to any one of claims 1 to 4, wherein the hydrophilic substance is one or more substances selected from the group consisting of polyethylene glycol, polyvinylpyrrolidone, polyvinyl alcohol and sodium alginate.

7. The electrode according to any one of claims 1 to 6, wherein the hydrophilic substance is cross-linked.

8. The electrode according to any one of claims 1 to 7, wherein the amount of the hydrophilic substance in the glucose diffusion layer is 1.19 µg/cm² or more and 1.19 g/cm² or less.

9. The electrode according to any one of claims 1 to 8, wherein the glucose diffusion layer has a thickness of 10 nm or more and 10 mm or less.

10. The electrode according to any one of claims 1 to 9, wherein the glucose diffusion layer covers the entire surface of the enzyme electrode layer.

11. The electrode according to any one of claims 1 to 9, wherein the glucose diffusion layer is provided only on the enzyme electrode layer.

12. The electrode according to any one of claims 1 to 11, wherein the permeation restricting layer comprises one or more components selected from the group consisting of a cellulose polymer, polyurethane and polyvinyl pyridine.

13. A glucose sensor comprising the electrode according to any one of claims 1 to 12.

14. Use of the glucose sensor according to claim 13 for continuous glucose monitoring.

15. A method of producing an electrode, the method comprising the steps of:
forming an enzyme electrode layer comprising an electrode material and a glucose oxidoreductase;
laminating a glucose diffusion layer comprising a hydrophilic substance on the enzyme electrode layer; and
laminating a permeation restricting layer on the glucose diffusion layer.
